# EUROPEAN PATENT APPLICATION

(11) **EP 4 293 014 A1**
(43) Date of publication of application: **20.12.2023**
(21) Application number: 22179089.2
(22) Date of filing: 15.06.2022
(51) Int. Cl.: C07D 213/61, C07B 39/00, C07D 213/73

(54) **A PROCESS FOR THE PHOTOLYTIC CHLORINATION OF HALOGENATED PYRIDINES WITH MOLECULAR CHLORINE**

(71) Applicant: BASF SE, 67056 Ludwigshafen am Rhein (DE)
(72) Inventor: Rack, Michael, 67056 Ludwigshafen an Rhein (DE); Kaduskar, Rahul, 400705 Navi Mumbai (IN); Gockel, Birgit, 67056 Ludwigshafen an Rhein (DE); Knoll, Daniel Maximilian, 67056 Ludwigshafen an Rhein (DE); Goetz, Roland, 67056 Ludwigshafen an Rhein (DE); Shinde, Harish, 400705 Navi Mumbai (IN)
(74) Representative: BASF IP Association

(57) **Abstract**

The present invention relates to a process for the chlorination of halogenated pyridines with molecular chlorine in the presence of water under UV light irradiation in the vapor phase. The present invention further relates to a multi-step process for producing 5-chloro-3,6-difluoro-pyridin-2-amine and to the novel intermediate compounds 2,3-dichloro-5,6-difluoropyridine and 2,5-dichloro-3,6-difluoro-pyridine, which can be obtained by the process of the present invention starting form 5-chloro-2,3-difluoropyridine and 3-chloro-2,5-difluoro-pyridine.

## Description

The present invention relates to a process for the chlorination of halogenated pyridines with molecular chlorine in the presence of water under UV light irradiation in the vapor phase. The present invention further relates to a multi-step process for producing 5-chloro-3,6-difluoropyridin-2-amine (IV) and to the novel intermediate compounds 2,3-dichloro-5,6-difluoropyridine (DCDFP) and 2,5-dichloro-3,6-difluoro-pyridine, which can be obtained by the process of the present invention starting from commercially available precursors.

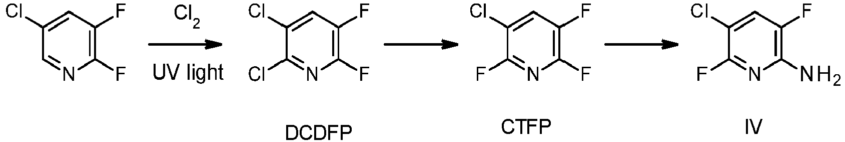

Compound IV is a key intermediate and building block in the synthesis of uracil pyridines, which were described in WO 2017/202768 as herbicides.

Substituted pyridines are highly desirable intermediates in chemical syntheses, especially in life sciences, for example in the pharmaceutical or agrochemical industry. Hence, there is a constant demand for versatile and efficient procedures, which enable the direct functionalisation of the pyridine ring with halogen substituents. Such transformations are of particular interest for industrial scale processes, which, for economic reasons, ask for cheap and readily available reactants such as molecular chlorine.

Pyridine is generally known for its poor reactivity towards molecular chlorine or other electrophiles like bromine or nitric acid in aromatic electrophilic substitution reactions. This behaviour is caused by its relative electron deficiency as compared to, for example, the unsubstituted or π-donor-substituted (Organikum, 20. Auflage, 1996, S. 337-340) benzene ring. Its reactivity is further reduced in the presence of electron-withdrawing substituents attached to the pyridine ring, comparable with nitro- or 1,3-dinitrobenzene. This chemical behaviour has prompted chemists to resort to other, non-conventional procedures in order to gain access to these structures: J. Fluorine Chem. 2000, 101, 45-60 discloses a multi-step approach, which is outlined in the scheme below, towards tetra-halogenated pyridines with substituent patterns matching those in intermediate CTFP (3-chloro-2,5,6-trifluoropyridine).

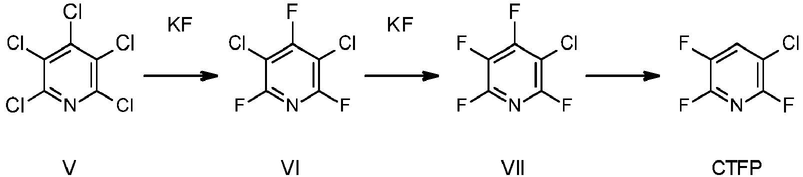

Accordingly, chemoselective fluorination of pentachloropyridine V with potassium fluoride aiming at the production of compound VII required two steps. Desymmetrization of intermediate VI was conducted in the presence of a catalyst. Yields for this second step reportedly were poor (65%, WO 1999/0044724) and it was established that purification of the reaction mixture implies the tedious recovery of unreacted compound VI, for example by distillation (Thesis F.G. Drakesmith, 1965, Durham University). The two-step halogen exchange was then followed by a hydro defluorination of compound VII with sodium borohydride (Chem. Eur. J. 2005, 11, 6, 1903-10) or Zn/NH₃ (US 2006047124), to obtain CTFP. The starting material V and the reactants used in hydro defluorinations are expensive, the transformations lack atom-efficiency and are not easily adaptable to large-scale industrial procedures. The process of the present invention offers a much more versatile approach, starting from less complex precursor molecules in a regioselective and very efficient manner, using molecular chlorine, making the process amenable to industrial processes.

Direct chlorination of the pyridine core using molecular chlorine was described in literature. For example, thermal procedures were developed for the chlorination of (unsubstituted) pyridine in the vapor phase at temperatures of about 400°C yielding mixtures of 2-chloropyridine and 2,6-dichloropyridine in poor to moderate yields (see for example US 3899495). Processes at these temperatures reportedly suffer from the formation of side products, tarring and/or corrosion of the reaction vessels.

The authors in US 3297556 report on a process to overcome these disadvantages by employing photolytic conditions in the liquid or vapor phase. The use of UV light irradiation enabled the transformation at considerably lower temperatures (at about 80 to 125°C), providing 2-chloropyridine in poor to moderate yields.

With reference to these procedures US 5536376 teaches that photolytic conditions in the vapor phase might be employed at higher temperatures than previously reported, i.e. up to 300°C, provided the chlorine and/or pyridine is sufficiently diluted with water (molar ratio of pyridine:water is from 1:10 to 1:30) to prevent overheating, which causes unwanted side reactions. The authors explicitly state, however, that unwanted side reactions pose a problem above 300°C. In this manner the authors were able to obtain mixtures of 2-chloropyridine and 2,6-dichloropyridine in moderate yields.

From an economic point of view, it is desirable in industrial large-scale operations to utilize highly selective, high yielding, inexpensive, simple and robust processes. In view of this, it was an object of the present invention to overcome the disadvantages of the reported procedures, providing a more efficient process for the chlorination of electron-deficient pyridines with high space-time yield and avoiding the formation of side products.

The inventors unexpectedly found that, in cases where the starting material are halogenated pyridines of formula I as defined herein, excellent conversion rates and high yields of regioselectively chlorinated pyridines can be achieved according to the process of the present invention. This finding is surprising because a skilled person would have expected a sluggish reaction with molecular chlorine considering the electron-deficient nature of the heteroaromatic core in compounds I. The inventors also found that the process is high-yielding particularly at temperatures above 300°C, with minimal amounts of side-products formed. This is in sharp contrast to the teaching of the prior art, which asserts that under such conditions intolerable amounts of side products will be formed.

Accordingly, the present invention relates to a process for the chlorination of halogenated pyridines of formula I, 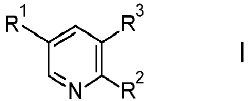 wherein
R¹ is hydrogen, chlorine, or fluorine;
R² is hydrogen, chlorine, or fluorine;
R³ is chlorine or fluorine;
with the exception of compounds I, wherein R¹ and R² are hydrogen;
the process comprising reacting compounds I in the presence of water and molecular chlorine under UV light irradiation and at a temperature above 290°C in the vapor phase; to obtain compounds of formula II, 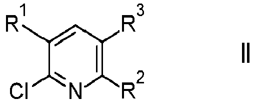 wherein, if R² is hydrogen in compounds I, R² in compounds II is chlorine.

The general expression "compound I" as used herein is equivalent to the expression "compound of formula I".

The chlorination reaction is conducted in the presence of a light source. Generally radiant energy sources covering, at least to some extent, the range between 2000 and 6000 angstrom units are effective to bring about the chlorinations of the present invention. Suitable light sources are ultraviolet light, black fluorescent light, mercury vapor light.

In one embodiment of the present invention R¹, R² and R³ are independently chlorine or fluorine in both, compounds I and II.

In one embodiment R¹ is hydrogen; and R², R³ are independently chlorine or fluorine in both, compounds I and II.

In another embodiment R¹, R³ are independently chlorine or fluorine; and R² is hydrogen in both, compounds I and II.

In a preferred embodiment R¹ is chlorine; and R², R³ are fluorine in both, compounds I and II.

The process of the present invention is typically carried out at atmospheric pressure. The reaction may also be conducted at elevated pressure, for example up to 2000 kPa.

In one aspect of the process of the present invention the temperature of the reaction mixture is above 300°C.

In one aspect of the process of the present invention the temperature of the reaction mixture is in the range between 300 and 450°C.

In yet another aspect the temperature of the reaction mixture is in the range between 300 and 380°C.

In one aspect of the process of the present invention the amount of water is more than 1 and less than 30 equivalents, based on the amount of compound I.

For optimal yields the inventors found that the molar ratio of pyridine:water in the reaction mixture should be less than 1:10. Therefore, in another aspect the amount of water is more than 1 and less than 10 equivalents, based on the amount of compound I.

In still another aspect the amount of water is more than 1 and less than 8 equivalents, based on the amount of compound I.

In still another aspect the amount of water is more than 3 and less than 10 equivalents, based on the amount of compound I.

In still another aspect the amount of water is more than 3 and less than 8 equivalents, based on the amount of compound I.

In one aspect of the process of the present invention the amount of molecular chlorine is 1-5 equivalents, based on the amount of compound I.

In a preferred aspect of the process of the present invention the amount of molecular chlorine is 1.5-2.5 equivalents, based on the amount of compound I.

In a preferred embodiment of the present invention the temperature of the reaction mixture is in the range between 300 and 450°C; and the amount of molecular chlorine is 1-5 equivalents, based on the amount of compound I.

In another preferred embodiment of the present invention the temperature of the reaction mixture is above 300°C; and the amount of molecular chlorine is 1-5 equivalents, based on the amount of compound I; and the amount of water is more than 1 and less than 10 equivalents, based on the amount of compound I.

In another preferred embodiment of the present invention the temperature of the reaction mixture is in the range between 300 and 450°C; and the amount of molecular chlorine is 1-5 equivalents, based on the amount of compound I; and the amount of water is more than 1 and less than 10 equivalents, based on the amount of compound I.

In a preferred aspect of the present invention the temperature of the reaction mixture is in the range between 300 and 400°C; and the amount of molecular chlorine is 1.5-2.5 equivalents, based on the amount of compound I; and the amount of water is more than 1 and less than 10 equivalents, based on the amount of compound I.

In another preferred embodiment of the present invention the temperature of the reaction mixture is in the range between 300 and 380°C; and the amount of molecular chlorine is 1.5-2.5 equivalents, based on the amount of compound I; and the amount of water is more than 1 and less than 10 equivalents, based on the amount of compound I.

In another preferred embodiment of the present invention the temperature of the reaction mixture is in the range between 300 and 380°C; and the amount of molecular chlorine is 1.5-2.5 equivalents, based on the amount of compound I; and the amount of water is more than 1 and less than 8 equivalents, based on the amount of compound I.

In another preferred embodiment of the present invention the temperature of the reaction mixture is in the range between 300 and 380°C; and the amount of molecular chlorine is 1.5-2.5 equivalents, based on the amount of compound I; and the amount of water is more than 3 and less than 8 equivalents, based on the amount of compound I.

In one aspect the present invention relates to a process as defined above, further comprising the step of reacting compound II, wherein R¹ is chlorine and R², R³ are fluorine, with a source of fluoride to obtain compound of formula III

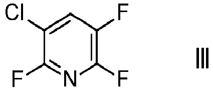

Typical sources of fluoride are, for example, potassium fluoride, potassium fluoride in mixture with hydrogen fluoride, sodium fluoride, calcium difluoride, Olah reagent (HF/pyridine), a polyhydrofluoride complex of a trialkylamine ((C₁-C₆-alkyl)₃N x n HF, wherein (n = 1-3) such as: (C₂H₅)₃N x 3 HF). Preferably, the fluorinating agent is used in an amount of 2.0 eq. to 10 eq., in particular 2.5-8.0 eq., more specifically 3.0-6.0 eq., based on the amount of compound II. According to a preferred embodiment the fluorinating reaction is carried out in the presence of an amine. Suitable amines are tertiary amines for example, tri(C₁-C₆-alkyl)amine such as trimethylamine, triethylamine or diisopropylethylamine, N-methylpiperidine, pyridine, substituted pyridines, such as 2,4,6-trimethylpyridine, 2,6-dimethylpyridine, 2-methylpyridine, 3-methylpyridine, 4-methylpyridine, and 4-dimethylaminopyridine; and also bicyclic amines such as 1,4-diazabicyclo[2.2.2]octane, 1,8-diazabicyclo[5.4.0]undec-7-ene, or 1,5-diaza-bicyclo[4.3.0]non-5-ene. Particular preference is given to using triethylamine, pyridine or 1,8-diazabicyclo[5.4.0]undec-7-ene.

In one aspect the fluorinating reaction is carried out in the presence of a phase transfer catalysts. Phase transfer catalysts suitable for use in the fluorination process are those well known in the art. Preferred phase transfer catalysts are selected from quaternary ammonium salts, quaternary pyridinium salts, quaternary phosphonium salts and any combination thereof and more preferably selected from quaternary ammonium salts, quaternary phosphonium salts and any combination thereof.

The fluorinating reaction is typically carried out in the presence of a polar solvent. The use of catalytic amounts of a polar solvent accelerates the fluorination reaction. Suitable organic solvents for the fluorination reaction are , aprotic polar solvents, for example: cyclic or acyclic ethers such as diethyl ether, diisopropyl ether, n-butyl methyl ether, isobutyl methyl ether, secbutyl methyl ether, tert-butyl methyl ether, cyclopentyl methyl ether, methyl-tetrahydrofuran, tetrahydrofuran, 1,4-dioxane, ethylene glycol dimethyl ether, ethylene glycol, diethyl ether, diethylene glycol dimethyl ether, 2-methyltetrahydrofuran, glycol monomethyl- or monoethyl ether; cyclic or acyclic amides such as carboxamides (N,N-dimethylformamide, N,N-dimethylacetamide, N,N-diethylformamide, N,N-diethylacetamide, N-methylpyrrolidone, dimethyl imidazolinum, dimethylimidazolidinone, or tetramethylurea; or aliphatic nitriles such as acetonitrile or propionitrile, and mixtures of the aforementioned solvents. N-substituted lactams, sulphoxides, sulphones (dimethyl sulfoxide, dimethyl sulfone, tetramethylene sulfoxide, tetramethylene sulfone, sulfolane). Preference is given to N,N-dimethylformamide, N-methylpyrrolidone , dimethylimidazolidinone , sulfolane. Preferably the polar solvent is sulfolane.

Typical reaction times for these fluorinations are in the range of from 1 to 24 hours, for example 4 to 12 hours. The reaction of the halogen exchange may be carried out at temperatures above 70°C, typically between 70°C and 180°C.

In one aspect the present invention relates to a process as defined above, further comprising the step of reacting compound III with aqueous or gaseous ammonia in at least stoichiometric amounts in a mixture with or without organic solvents like ethers, alcohols, aromatic hydrocarbons, dipolar aprotic solvents such as those defined above for fluorination reactions to obtain compound of formula IV.

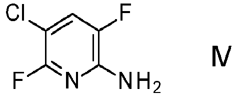

Aqueous ammonia is particularly preferred in industrial set-ups. It may be advantageous to conduct these reactions under pressure, for example in the range between 100 and 2000 kPa, preferably between 400 and 600 kPa. Typical reaction times for these aminations are in the range of from 1 to 24 hours, for example 4 to 8 hours. The reaction of the halogen exchange may be carried out at temperatures between 25 and 200°C, typically between 80°C and 150°C, preferably between 90 and 140°C.

In one aspect the present invention further relates to the novel intermediate compound of formula II, wherein R¹ is chlorine; and wherein R² and R³ are fluorine (DCDFP).

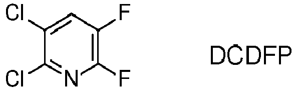

In one aspect the present invention further relates to the novel intermediate compound of formula II, wherein R¹ and R² are fluorine; and wherein R³ is chlorine.

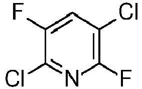

### Working Examples

The present invention is further illustrated by the following working examples.

### Example 1.1) Chlorination of 5-chloro-2,3-difluoropyridine

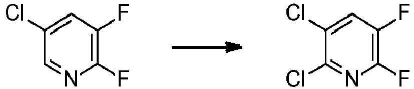

General protocol: in a glass gas phase reactor with a length of 58 cm and a diameter of 3 cm equipped with a thermometer and two inlets for the gaseous starting material and the chlorine gas. A gaseous mixture of compounds I and water was injected with a flow rate of 0,1 mL/minute. At the same time molecular chlorine was introduced to the reactor with a flow rate of 2.5 L/hour The mean residence time of the reaction mixture in the reactor is estimated at 2-4 seconds. The reaction was carried out under UV light irradiation (mercury vapor UV lamp: Heraeus TQ 150, Z 3 doped, radiation flux Φ 200-600 nm, 50 W). The temperature in the reaction zone was maintained by means of the heat of the UV lamp.

After the reaction was complete the reactor was purged with nitrogen and cooled down. The crude reaction mixture was collected as a liquid, which was washed with 40% aqueous sodium hydroxide solution and distilled under reduced pressure (95°C/10 kPa). The conversion and purity of the title product 2,3-dichloro-5,6-difluoropyridine was determined by quantitative gas chromatography (GC).

Table I summarizes the results obtained in accordance with the protocol of Example 1.1) under different reaction conditions.

**Table I**

| Entry | Temperature of reaction mixture [°C] | Amount of chlorine based on amount of pyridine [equiv.] | Molar ratio pyridine : water | Conversion [%] | Isolated yield [%] |
|---|---|---|---|---|---|
| #1^{a)} | 270 | 2 | 1 : 6 | 61 | 41 |
| #2^{a)} | 290 | 2 | 1 : 6 | 71 | 46 |
| #3 | 320 | 2 | 1 : 3 | 76 | nd |
| #4 | 320 | 2 | 1 : 3 | nd | 70 |
| #5 | 320 | 2 | 1 : 6 | 91 | 79.6* |
| #6 | 350 | 2 | 1 : 6 | 92.6 | 81.4 |
| #7 | 350 | 2 | 1 : 10 | 81.8 | 81.4 |

| | | | | | |
|---|---|---|---|---|---|
| ^{a)} example not according to invention *estimated based on quantitative GC | | | | | |

### Example 1.2) Chlorination of 3-chloro-2,5-difluoro-pyridine

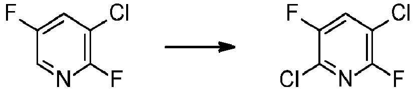

3-Chloro-2,5-difluoro-pyridine was chlorinated as described in Example 1.1, entry #6. The compound was not distilled. The conversion was 92.2% and purity of the title product 2,5-dichloro-3,6-difluoro-pyridine was determined by quantitative gas chromatography; yield: 89.8%.

### Example 2) Fluorination of 2,3-dichloro-5,6-difluoropyridine

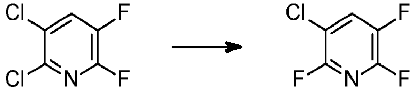

11.6 g (0.2 mol) potassium fluoride (spray dried) and 0.37 g (0.01 mol) *tetrakis*(1-piperidyl)phosphonium chloride were suspended in 75.4 g (0.63 mol) sulfolane. After that, 12 g sulfolane was distilled off under reduced pressure (0.3 mbar; 140°C). After cooling to 25°C 19.95 g of 2,3-dichloro-5,6-difluoropyridine (0.1 mol; purity according to GC: 92.7%) was added to the reaction mixture and stirred for 12 hours at 149°C and 2 hours at 157°C. Thereafter, the product was distilled off under reduced pressure (130-160°C oil bath temperature; 100-15 mbar). 15.05 g of 92% pure 3-chloro-2,5,6-trifluoropyridine was isolated; 82.2% yield. By using octyl(triphenyl)phosphonium bromide as catalyst a yield of 80.0% and by using of 1-benzyl-N,N-dimethyl-pyridin-1-ium-4-amine chloride as catalyst a yield of 80.0% was achieved.

### Example 3) Amination of 3-chloro-2,5,6-trifluoropyridine

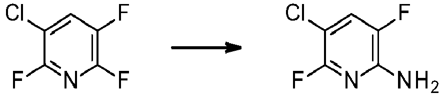

5 g (0.27 mol) 3-chloro-2,5,6-trifluoropyridine (98%) was placed in a pressure reactor and 135 g (1.98 mol) aqueous ammonia (25%) was added, the reactor was closed and heated up to 120°C for 4 hours. After cooling to 25°C the pressure was released, the product extracted with 600 mL dichloromethane. After phase separation the organic phase was washed three times with 200 mL water, dried with magnesium sulfate and evaporated under reduced pressure. 40.0 g of 5-chloro-3,6-difluoropyridin-2-amine was isolated (84% yield, 95% purity). The product contained 5% of the isomeric compound 3-chloro-5,6-difluoro-pyridin-2-amine.

### GC analytics:

Quantitative and qualitative method: solvent acetonitrile, 1-decanol reference item

| | | | |
|---|---|---|---|
| Column | HP-5, 30m x 0.32mm, 0.25 µm | | |
| Injector temperature | 280°C | | |
| FID temperature | 320°C | | |
| Oven temperature | Rate [°C/min] | Value [°C] | Hold Time [min] |
| | - | 60 | 0 |
| | 15 | 280 | 10 |
| Carrier gas | hydrogen | | |
| Detector | FID | | |
| Split ratio | 50:1 | | |
| Column flow | 0.5 mL/minute (constant flow) | | |
| Injection volume | 1 µL | | |
| Analysis time | 24.7 min | | |

| Target compound | Retention time [min] |
|---|---|
| 2,3-dichloro-5,6-difluoropyridine | 8.63 |
| 1-decanol | 10.79 |

## Claims

1. A process for the chlorination of halogenated pyridines of formula I, 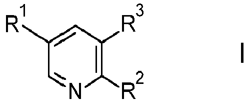 wherein
R¹ is hydrogen, chlorine, or fluorine;
R² is hydrogen, chlorine, or fluorine;
R³ is chlorine or fluorine;
with the exception of compounds I, wherein R¹ and R² are hydrogen;
the process comprising reacting compounds I in the presence of water and molecular chlorine under UV light irradiation and at a temperature above 290°C in the vapor phase; to obtain compounds of formula II, 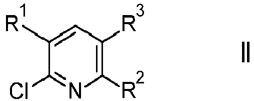 wherein, if R² is hydrogen in compounds I, R² in compounds II is chlorine.

2. A process according to claim 1, wherein the temperature is in the range between 300 and 450°C.

3. A process according to claim 1 or 2, wherein the amount of chlorine is 1-5 equivalents, based on the amount of compound I.

4. A process according to any one of claims 1 to 3, wherein the amount of water is between 1 and 30 equivalents, based on the amount of compound I.

5. A process according to any one of claims 1 to 3, wherein the amount of water is more than 1 and less than 10 equivalents, based on the amount of compound I.

6. A process according to any one of claims 1 to 5, wherein R¹, R² and R³ are independently chlorine or fluorine in both, compounds I and II.

7. A process according to any one of claims 1 to 5, wherein R¹ is chlorine; and wherein R², R³ are fluorine in both, compounds I and II.

8. A process according to claim 7, further comprising the step of reacting compound II with a source of fluoride to obtain compound of formula III

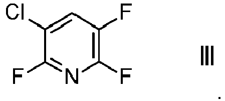

9. A process according to claim 8, further comprising the step of reacting compound III with ammonia to obtain compound of formula IV

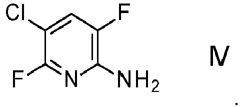

10. An intermediate compound of formula II, wherein R¹ is chlorine; and wherein R², R³ are fluorine.

11. An intermediate compound of formula II, wherein R¹ and R² are fluorine; and wherein R³ is chlorine.
